# EUROPÄISCHE PATENTSCHRIFT

(11) **EP 3 027 584 B1**
(45) Veröffentlichungstag und Bekanntmachung des Hinweises auf die Patenterteilung: **14.06.2017**
(21) Anmeldenummer: 14761946.4
(22) Anmeldetag: 25.07.2014
(51) Int. Cl.: C07C 37/045, C07C 51/367

(54) **VERFAHREN ZUR HERSTELLUNG VON PHENOLEN**
METHOD FOR THE PREPARATION OF PHENOLS
PROCÉDÉ DESTINÉ À LA FABRICATION DE PHÉNOLS

(30) Priorität: 27.07.2013 EP 13003758
(43) Veröffentlichungstag der Anmeldung: 08.06.2016
(73) Patentinhaber: WeylChem Frankfurt GmbH, 65933 Frankfurt Griesheim (DE)
(72) Erfinder: FORSTINGER, Klaus, 64832 Babenhausen (DE); SCHWESINGER, Reinhard, 79249 Merzhausen (DE); MAIER, Andreas, 65817 Eppstein (DE)
(74) Vertreter: Zellentin & Partner mbB Patentanwälte
(86) Internationale Anmeldenummer: PCT/EP2014/002033
(87) Internationale Veröffentlichungsnummer: WO 2015/014464

(56) Entgegenhaltungen:
- CN-A- 102 199 073
- CN-A- 102 746 122
- US-A- 5 416 235

## Beschreibung

Die vorliegende Erfindung betrifft ein Verfahren zur Herstellung von Phenolen aus aromatischen Aminen.

Es ist bekannt, dass Phenole durch Verkochung von Diazoniumsalzen, welche durch die Diazotierung von aromatischen Aminoverbindungen mittels salpetriger Säure in der Kälte entstehen, hergestellt werden können. Gemäß "Ullmann's Encyclopedia of Industrial Chemistry" (7th Completely Revised Edition, vol. 11, p. 279) wird die Diazotierung und die Verkochung am besten in Schwefelsäure-Lösung durchgeführt. Es besteht jedoch die Problematik, dass bei der einfachsten Methode wie dem Erhitzen des Diazoniumsalzes auf bis zu 80-100 °C das erhaltene Produkt eine niedrige Reinheit aufweist. Eine Erhöhung der Ausbeute und Selektivität kann manchmal durch das Entfernen des gebildeten Phenols mittels aufwändiger, gleichzeitiger Dampfdestillation aus der Reaktionsmischung (s. z.B. DE 2 426 994 A1) oder durch das Lösen des entstehenden Phenols in Xylol als organisches Lösungsmittel erzielt werden.

Aus US 1910679 ist ein Verfahren zur Herstellung von Phenolen bekannt, bei welchem die Verkochung von entsprechenden Diazoniumsalzen in einer wässrigen sauren Lösung in Gegenwart eines organischen Lösungsmittels durchgeführt wird, wobei Anisol, Xylol oder Chlorbenzol als organische Lösungsmittel verwendet wurden. Jedoch ist die Ausbeute in diesem Verfahren nicht zufriedenstellend.

Die US 5,416,235 A und die CN 102746122 A beschreiben Verfahren zur Umsetzung von aromatischen Aminen zu Phenolen durch Diazotierung und anschließende Verkochung in Anwesenheit eines Lösungsmittels sowie von Kupfersalzen. In den Beispielen werden Ethylacetat bzw. Butylacetat oder Methylisobutylketon als Lösemittel verwendet.

Gemäß CN 102199073 A lässt sich 4,4'-Dihydroxydiphenylmethan durch Diazotierung und Verkochung von 4,4'-Diaminodiphenylmethan erhalten, wenn die Verkochung in Wasser und einem nicht mit Wasser mischbaren Lösungsmittel erfolgt.

Die Aufgabe der Erfindung besteht darin, ein Verfahren zur Herstellung von Phenolen bereit zu stellen, durch das eine höhere Selektivität und Ausbeute von Phenolen bei deren Herstellung aus entsprechenden aromatischen primären Aminen durch die Diazoverkochung erzielt werden kann.

Überraschend wurde nun gefunden, dass eine Erhöhung der Selektivität und Ausbeute durch die Verwendung von aliphatischen Ketonen als Lösungsmittel bei der Diazoverkochung erzielt werden kann. Diese Ketone sind im Wesentlichen schlecht mit einem wässrigen Medium mischbar, in dem eine entsprechende Diazo-Verbindung einer Zersetzung unterzogen wird.

Der Gegenstand der vorliegenden Erfindung ist somit ein Verfahren zur Herstellung von Phenolen, bei dem ein Aryldiazoniumsalz, das durch die Diazotierung eines entsprechenden aromatischen, primären Amins, ausgewählt unter Anilin und substituiertem Anilin, das mindestens einen weiteren Substituenten enthält, der ausgewählt ist aus: Alkyl, Alkenyl, Alkinyl, Halogen, Haloalkyl, Cycloalkyl, Heteroaryl, Carboxyl, Cyan, Alkoxyl und Ester, hergestellt wird, durch Erhitzen in einem heißes Wasser und eine Mineralsäure sowie ein organisches Lösungsmittel enthaltenden Gemisch zum Phenol umgesetzt wird, wobei das organische Lösungsmittel ein Keton der Formel (I) R¹C(O)R² 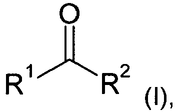 enthält, in der R¹ und R² unabhängig voneinander für (C₁-C₅)-Alkyl stehen und R¹ und R² zusammen mindestens vier Kohlenstoffatome aufweisen und wobei die

Lösung bei der Umsetzung zum Phenol im wesentlichen keine Kupfersalze enthält.

Im erfindungsgemäßen Verfahren erhält man überraschenderweise eine sehr hohe Selektivität im Vergleich zu Verfahren, bei denen entweder kein organisches Lösungsmittel oder Lösungsmittel wie Xylol, Chlorbenzol, Toluol, Methyl-*tert*-butylether (MTBE), Essigsäure-n-Butylester usw. verwendet werden. Außerdem kann durch das erfindungsgemäße Verfahren eine höhere Ausbeute erzielt werden.

In einer bevorzugten Ausführungsform des erfindungsgemäßen Verfahrens können R¹ und R² in der Verbindung der Formel (I) unabhängig voneinander folgende Bedeutung aufweisen: Methyl, Ethyl, *n*-Propyl, *iso*-Propyl, *n-*Butyl, *iso*-Butyl, *tert-*Butyl, *n*-Pentyl, *iso*-Pentyl und *tert*-Pentyl. Bevorzugt sind die Verbindungen, bei denen R¹ für Methyl oder Ethyl und R² für Ethyl oder *iso*-Butyl stehen. Insbesondere sind die Verbindungen der Formel (I) bevorzugt, in denen R¹ für Methyl und R² für *iso*-Butyl (Methylisobutylketon) oder R¹ und R² für Ethyl (3-Pentanon) stehen. Ganz besonders ist Methylisobutylketon als Verbindung der Formel (I) bevorzugt.

Die Diazotierungsreaktion wird in an sich bekannter Weise durchgeführt (s. z.B. "Ullmann's Encyclopedia of Industrial Chemistry", 7th Completely Revised Edition, vol. 11, p. 279). Das aromatische Amin wird üblicherweise in einem wässrigen Medium in Gegenwart einer Mineralsäure bei einer Temperatur von etwa -5 bis etwa 50 °C, vorzugsweise von etwa 0 bis etwa 20 °C, in Gegenwart eines zur Diazotierung geeigneten Reagenzes, bevorzugt in Gegenwart von salpeteriger Säure, diazotiert.

Die eingesetzten Amine umfassen Anilin und substituierte Aniline, die mindestens einen weiteren Substituenten enthalten, der ausgewählt ist aus: Alkyl, Alkenyl, Alkinyl, Halogen, Haloalkyl, Cycloalkyl, Heteroaryl, Carboxyl, Cyan, Alkoxyl und Ester. Bevorzugt werden folgende Gruppen: (C₁-C₆)-Alkyl, (C₂-C₆)-Alkenyl, (C₂-C₆)-Alkinyl, Fluor, Chlor, Brom, Iod, (C₁-C₃)-Haloalkyl, (C₃-C₆)-Cycloalkyl, Heteroaryl, Carboxyl und Ester. Besonders bevorzugt sind primäre aromatische Amine, die Methyl-, Ethyl-, Fluor-, Chlor-, Brom-, lod- und/oder Carboxyl-Gruppen aufweisen.

Das Diazotiermittel wird im erfindungsgemäßen Verfahren im Allgemeinen in einer stöchiometrischen Menge oder einem kleinen Überschuss verwendet. Geeignet ist ein molares Verhältnis von Diazotiermittel zu aromatischem Amin von etwa 1 : 1 bis etwa 1,5 : 1, bevorzugt von etwa 1 : 1 bis etwa 1,1 : 1.

Als Diazotiermittel können z.B. Alkalinitrite, wie Natrium- oder Kaliumnitrit, Distickstofftrioxid und salpetrige Säure, Nitrosylhalogenide, wie Nitrosylchlorid oder -bromid, und andere Nitrosylsalze, wie Nitrosylperchlorat, - tetrafluorborat oder -sulfat verwendet werden. Natriumnitrit, Kaliumnitrit, Distickstofftrioxid oder salpetrige Säure werden bevorzugt, wobei Natriumnitrit besonders bevorzugt wird.

Die bei der Diazotierungsreaktion verwendete Mineralsäure hat vorzugsweise eine Konzentration von etwa 20 bis etwa 98 %. Besonders bevorzugt ist eine Konzentration von etwa 20 bis etwa 50 % oder von etwa 95 bis etwa 97 %. In einer bevorzugten Ausführungsform der Erfindung kann Schwefelsäure, Phosphorsäure, HCl oder deren Gemische als Mineralsäure bei der Diazotierungsreaktion verwendet werden. Schwefelsäure wird besonders bevorzugt.

Das gebildete Diazoniumsalz wird im Allgemeinen bei einer Temperatur von etwa 70 bis etwa 120 °C, vorzugsweise etwa 80 bis etwa 100 °C zersetzt (Verkochung). Vorzugsweise wird bei der Zersetzung das das Diazoniumsalz enthaltende Gemisch langsam einer Mischung aus einem Lösungsmittel der allgemeinen Formel (I), heißem Wasser und einer Mineralsäure zudosiert. Diese Lösung enthält im wesentlichen keine Kupfersalze, das heißt es werden keine Kupfersalze (oder Kupfer) zugefügt. Sehr geringe Mengen Kupfersalze, welche als Verunreinigung in den verwendeten Substanzen vorhanden sein könnten, stören nicht.

Typischerweise ist ein Mengenverhältnis des organischen Lösungsmittels zur wässrigen Mineralsäure von etwa 1:100 bis etwa 100:1, bevorzugt von etwa 1:25 bis etwa 25:1 geeignet. Die Konzentration der bei der Zersetzung verwendeten Mineralsäure kann im Bereich von etwa 10 bis etwa 98 %, bevorzugt etwa 20 bis etwa 96 %, liegen.

In einer bevorzugten Ausführungsform der Erfindung wird Schwefelsäure oder ein Gemisch aus Schwefelsäure und Phosphorsäure als Mineralsäure bei der Diazoverkochung verwendet. Schwefelsäure ist besonders bevorzugt.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

### Beispiel 1

In einem Rührkolben mit Thermometer, Rührer und Tropftrichter wurden 500 mL 24%ige Schwefelsäure vorgelegt. Dazu wurden 68 g Anthranilsäure gegeben. Diese Ausgangsmischung wurde bei 0°C bis 3 °C mit 87,8 g 40%iger Natriumnitrit-Lösung diazotiert. Die Diazoniumsalzlösung wurde dann bei 98°C und guter Rührung in eine Mischung aus 25 mL 24%iger Schwefelsäure und 500 mL Methyl-iso-butylketon eindosiert. Nach ca. 30 min. Nachreaktion bei 98°C wurde nach Abkühlung der Emulsion auf ca. 85°C eine Phasentrennung vorgenommen. Die organische Phase wurde mit einer Mischung aus 750 mL Wasser und 54 g 50%iger Natronlauge extrahiert. Nach erneuter Phasentrennung wurde aus der unteren wässerigen Phase das Zielprodukt (2-Hydroxybenzoesäure) durch Ansäuerung mit Salzsäure (pH<1,5) ausgefällt. Die Isolierung des Endproduktes erfolgte durch Filtration. Nach Waschen bis zum neutralen pH-Wert des Waschwassers und Trocknung des Filterkuchens wurden 64,1 g 2-Hydroxybenzoesäure mit einem Gehalt von 99,6% a/a (gemäß HPLC) erhalten. Das entspricht einer Ausbeute von: 93,5%. Die Selektivität betrug nach der Verkochung: 97,7% a/a (mittels HPLC bestimmt).

### Vergleichsbeispiel 2

Die Einsatzmengen und die Durchführung der Synthese waren die gleichen wie bei Beispiel 1. Als organisches Lösungsmittel wurde jedoch Xylol verwendet.
Auswaage Produkt: 55,8 g
Gehalt: 97,3% a/a (HPLC)
Ausbeute: 79,3%.
Selektivität nach Verkochung: 94,2% a/a (HPLC).

### Beispiel 3

In einem Rührkolben mit Thermometer, Rührer und Tropftrichter wurden 700 mL 22%ige Schwefelsäure vorgelegt. Dazu wurden 70,8 g 5-Chlor 2-methylanilin gegeben. Nach Heizen auf ca. 90°C und anschließender Abkühlung auf 10°C-12°C wurde die erhaltene Suspension mit 88,4 g 40%iger Natriumnitrit-Lösung diazotiert. Die Diazoniumsalzlösung wurde dann bei ca. 85-90°C und guter Rührung in eine Mischung aus 200 mL Methyl-*iso-*butylketon und 2 g 96%iger Schwefelsäure eindosiert. Nach ca. 30 min. Nachreaktion bei 85-90°C wurde nach Abkühlung der Emulsion auf ca. 70°C eine Phasentrennung vorgenommen. Die organische Phase wurde nicht weiter aufgearbeitet (destilliert). Es wurde die Selektivität mittels GC-Analyse ermittelt. Die Selektivität nach Verkochung betrug: 99,4% a/a (GC).

### Vergleichsbeispiel 4

Die Einsatzmengen und die Durchführung der Synthese waren die gleichen wie bei Beispiel 3. Als organisches Lösungsmittel wurde jedoch Xylol verwendet (Verkochungstemperatur: ca. 90°C). Die Selektivität nach Verkochung betrug: 94,1 % a/a (GC).

### Beispiel 5

In einem Rührkolben mit Thermometer, Rührer und Tropftrichter wurden 700 mL 22%ige Schwefelsäure vorgelegt. Dazu wurden 70,8 g 3-Chlor-2-methylanilin gegeben. Nach Hochheizen auf ca. 90°C und anschließender Abkühlung auf 10°C-12°C wurde die vorliegende Suspension mit 88,4 g 40%iger Natriumnitrit-Lösung diazotiert. Die Diazoniumsalzmischung wurde dann bei ca. 90°C und guter Rührung in eine Mischung aus 200 mL Methyl-*iso*-butylketon und 2 g 96%iger Schwefelsäure eindosiert. Nach ca. 15 min. Nachreaktion bei 90°C wurde nach Abkühlung der Emulsion auf ca. 70°C eine Phasentrennung vorgenommen. Die organische Phase wurde noch einmal mit 50 mL Wasser gewaschen. Die Selektivität nach Verkochung betrug: 99,1% a/a (GC). Die organische Phase wurde dann destilliert. Es wurden 66,9 g 3-Chlor-2-methylphenol erhalten. Destillationsrückstand: 2,3 g. Der Gehalt des Zielproduktes betrug: 99,2% a/a (GC). Das entspricht einer Ausbeute von 93,1%.

### Beispiel 6

Die Einsatzmengen und die Durchführung der Synthese waren die gleichen wie bei Beispiel 5. Als organisches Lösungsmittel wurde jedoch 3-Pentanon verwendet. Die Selektivität nach Verkochung betrug: 96,9% a/a (GC).

### Vergleichsbeispiel 7

Die Einsatzmengen und die Durchführung der Synthese waren die gleichen wie bei Beispiel 5. Als organisches Lösungsmittel wurde jedoch Toluol verwendet. Die Selektivität nach Verkochung betrug: 83,0% a/a (GC). Endprodukt (destilliert): 58,7 g. Destillationsrückstand: 14,8 g. Der Gehalt des Zielproduktes betrug: 97,1% a/a (GC). Das entspricht einer Ausbeute von 80%.

### Beispiel 8

In einem Rührkolben mit Thermometer, Rührer und Tropftrichter wurden 375 mL 34%ige Schwefelsäure vorgelegt. Dazu wurden 60 g 3,4-Dimethylanilin gegeben. Nach Hochheizen auf ca.75°C und anschließender Abkühlung auf 0°C-3°C wurde die vorliegende Suspension mit 85,5 g 40%iger Natriumnitrit-Lösung diazotiert. Die Diazoniumsalzlösung wurde dann bei ca. 85°C und guter Rührung in eine Mischung aus 50 mL Methylisobutylketon und 120 mL 50%iger Schwefelsäure eindosiert. Nach ca. 30 min. Nachreaktion bei 85°C wurde, nach Abkühlung der Emulsion auf ca. 35°C, eine Phasentrennung vorgenommen. Die organische Phase wurde nicht weiter aufgearbeitet (destilliert). Es wurde die Selektivität mittels GC-Analyse ermittelt. Die Selektivität nach Verkochung betrug: 97,2% a/a (GC). Nach Abzug Lösemittel betrug die Ausbeute an Produkt 94,5 %.

### Vergleichsbeispiel 9

Die Einsatzmengen und die Durchführung der Synthese waren die gleichen wie bei Beispiel 8. Als organisches Lösungsmittel wurde jedoch Xylol verwendet. Die Selektivität nach Verkochung betrug: 93,1% a/a (GC). Nach Abzug Lösemittel betrug die Ausbeute an 3,4-Dimethylphenol 80,1 %.

### Vergleichsbeispiel 10

Die Einsatzmengen und die Durchführung der Synthese waren die gleichen wie bei Beispiel 8. Als organisches Lösungsmittel wurde jedoch Chlorbenzol verwendet. Die Selektivität nach Verkochung betrug: 94,2% a/a (GC). Nach Abzug Lösemittel betrug die Ausbeute an 3,4-Dimethylphenol 80,0 %

### Vergleichsbeispiel 11

Die Einsatzmengen und die Durchführung der Synthese waren die gleichen wie bei Beispiel 8. Bei der Verkochung wurde kein org. Lösungsmittel verwendet. Die Selektivität nach Verkochung betrug: 82,8% a/a (GC). Nach Abzug Lösemittel betrug die Ausbeute an 3,4-Dimethylphenol 59,2 %

### Vergleichsbeispiel 12

Die Einsatzmengen und die Durchführung der Synthese waren die gleichen wie bei Beispiel 8. Als organisches Lösungsmittel wurde jedoch Cyclohexanon verwendet. Die Selektivität nach Verkochung betrug: 80,2% a/a (GC). Die Ausbeute an 3,4-Dimethylphenol betrug nach Destillation 70,2 %.

### Vergleichsbeispiel 13

Die Einsatzmengen und die Durchführung der Synthese waren die gleichen wie bei Beispiel 8. Als organisches Lösungsmittel wurde jedoch Methylcyclohexan verwendet. Die Selektivität nach Verkochung betrug: 76,6 % a/a (GC). Nach Abzug Lösemittel betrug die Ausbeute an 3,4-Dimethylphenol 75,2%.

### Vergleichsbeispiel 14

Die Einsatzmengen und die Durchführung der Synthese waren die gleichen wie bei Beispiel 8. Als organisches Lösungsmittel wurde jedoch Ethylacetat verwendet. Die Selektivität nach Verkochung betrug: 83,9 % a/a (GC).

Wie die Ausführungsbeispiele zeigen, kann durch das erfindungsgemäße Verfahren eine deutliche Erhöhung der Selektivität und Ausbeute im Vergleich sowohl zu den bisher im Stand der Technik verwendeten organischen Lösungsmitteln wie Xylol oder Chlorbenzol als auch solchen Lösungsmitteln wie Toluol, Cyclohexanon oder Methylcyclohexan erzielt werden.

### Vergleich mit CN 102746122

Gemäß Beispiel 1 in CN 102746122 wurde in einem 1l Reaktor zu 408g 15 %-ige Schwefelsäure 27,8 g p-Aminophenol gegeben und vermischt. Bei 0°C-5°C wurden 48 g 40 %-ige Natriumnitritlösung innerhalb 1 h zugetropft. Vollständige Umsetzung zum Diazoniumsalz. Überschüssiges Natriumnitrit wurde mittels Amidosulfonsäure zerstört. Das erhaltene Gemisch wurde bei 88 bis 90 °C innerhalb 1 h in einen gerührten 2l Reaktor getropft, in der 300ml Wasser, 42,5 g basisches Kupfercarbonat, 143,5 g Kupfersulfatpentahydrat in Wasser und 200 g n-Butylacetat oder 200 g Methylisobutylketon oder 200 g Ethylacetat (abweichende Umsetzungstemperatur 70°C-73°C) vorgelegt waren. Nach 3 Stunden Rühren und Abkühlen auf 20 °C über Nacht wurde die Lösemittelphase abgetrennt, die wässrige Phase zweimal mit je 100 g des verwendeten Lösemittels extrahiert und Selektivität per HPLC bestimmt. Der Versuch einer Umsetzung in Xylol war erfolglos.

Die folgende Tabelle 1 zeigt die Ergebnisse.

**Tabelle 1**

| Lösemittel | Selektivität |
|---|---|
| MIBK | 76,6 % |
| Butylacetat | 74,9 % |
| Ethylacetat | 82,0 % |

Man erkennt dass die mit MIBK erzielten Selektivitäten vergleichbar mit n-Butylacetat und schlechter als mit Ethylacetat sind. Weiterhin ist festzustellen dass trotz des Einsatzes der hohen Kupfermengen die Selektivitäten noch nicht zufriedenstellend sind. Das Handling der großen Mengen Kupfersalze ist ohnehin schwierig, selbst wenn diese wie in CN 102746122 beschrieben aufgearbeitet und in den Prozess zurückgeführt werden können.

## Patentansprüche

1. Verfahren zur Herstellung von Phenolen, bei dem ein Aryldiazoniumsalz, das durch die Diazotierung eines entsprechenden aromatischen primären Amins hergestellt wird, durch Erhitzen in einem heißes Wasser, eine Mineralsäure und ein organisches Lösungsmittel enthaltenden Gemisch zersetzt wird,
**dadurch gekennzeichnet,**
**dass** das organische Lösungsmittel ein Keton der Formel (I) R¹C(O)R² 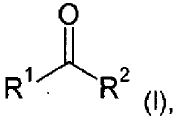 in der R¹ und R² unabhängig voneinander für (C₁-C₅)-Alkyl stehen und R¹ und R² zusammen mindestens vier Kohlenstoffatome aufweisen, enthält, wobei das aromatische primäre Amin Anilin oder ein substituiertes Anilin, das mindestens einen weiteren Substituenten enthält, der ausgewählt ist aus: Alkyl, Alkenyl, Alkinyl, Halogen, Haloalkyl, Cycloalkyl, Heteroaryl, Carboxyl, Cyan, Alkoxyl und Ester, ist und wobei in dem heißes Wasser, eine Mineralsäure und ein organisches Lösungsmittel enthaltenden Gemisch im wesentlichen keine Kupfersalze enthalten sind.

2. Verfahren nach Anspruch 1, **dadurch gekennzeichnet, dass** das Anilin mindestens einen weiteren Substituenten enthält, der ausgewählt ist aus: (C₁-C₆)-Alkyl, (C₂-C₆)-Alkenyl, (C₂-C₆)-Alkinyl, Fluor, Chlor, Brom, Iod, (C₁-C₃)-Haloalkyl, (C₃-C₆)-Cycloalkyl, Heteroaryl, Carboxyl und Ester.

3. Verfahren nach Anspruch 2, **dadurch gekennzeichnet, dass** der weitere Substituent ausgewählt ist aus: Methyl-, Ethyl-, Fluor-, Chlor-, Brom-, lod-, Carboxyl-Gruppen und zwei oder mehr davon.

4. Verfahren nach einem der Ansprüche 1 bis 3, **dadurch gekennzeichnet, dass** R¹ und R² unabhängig voneinander aus der Gruppe bestehend aus Methyl, Ethyl, *n*-Propyl, *iso*-Propyl, *n*-Butyl, *iso*-Butyl, *tert*-Butyl, *n*-Pentyl, *iso*-Pentyl, *tert*-Pentyl ausgewählt sind.

5. Verfahren nach Anspruch 4, **dadurch gekennzeichnet, dass** R¹ für Methyl oder Ethyl und R² für Ethyl oder *iso*-Butyl stehen, vorzugsweise R¹ für Methyl und R² für *iso*-Butyl oder R¹ und R² für Ethyl stehen.

6. Verfahren nach Anspruch 5, **dadurch gekennzeichnet, dass** Methylisobutylketon oder 3-Pentanon, bevorzugt Methylisobutylketon, als Keton der Formel (I) verwendet wird.

7. Verfahren nach einem oder mehreren der Ansprüche 1-6, **dadurch gekennzeichnet, dass** die Konzentration der Mineralsäure von etwa 10 bis etwa 98 %, bevorzugt von etwa 20 bis etwa 50 % oder von etwa 95 bis etwa 97 %, beträgt.

8. Verfahren nach einem oder mehreren der Ansprüche 1-7, **dadurch gekennzeichnet, dass** das Mengenverhältnis von organischem Lösungsmittel der Formel (I) zur wässrigen Lösung der Mineralsäure etwa 1:100 bis etwa 100:1, bevorzugt etwa 1:25 bis etwa 25:1 beträgt.

9. Verfahren nach einem oder mehreren der Ansprüche 1-8, **dadurch gekennzeichnet, dass** Schwefelsäure oder Phosphorsäure, bevorzugt Schwefelsäure, als Mineralsäure eingesetzt wird.

10. Verfahren nach einem oder mehreren der Ansprüche 1-9, **dadurch gekennzeichnet, dass** das Aryldiazoniumsalz bei einer Temperatur von etwa 70 bis etwa 120 °C, vorzugsweise von etwa 80 bis etwa 110 °C zersetzt wird.

## Claims

1. Method for the preparation of phenols in which an aryldiazonium salt, which is prepared by the diazotisation of a corresponding aromatic primary amine, is decomposed by heating in a mixture containing hot water, a mineral acid and an organic solvent,
**characterised in that,**
the organic solvent contains a ketone of formula (I) R¹C(O)R² 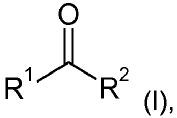 in which R¹ and R² independently stand for (C₁-C₅)-alkyl and R¹ and R² together have at least four carbon atoms, wherein the aromatic primary amine is aniline or a substituted aniline which contains at least one further substituent which is selected from: alkyl, alkenyl, alkynyl, halogen, haloalkyl, cycloalkyl, heteroaryl, carboxyl, cyanide, alkoxyl and ester, and wherein substantially no copper salts are contained in the mixture containing hot water, a mineral acid and an organic solvent.

2. Method according to claim 1, **characterised in that** the aniline contains at least one further substituent, which is selected from: (C₁-C₆)-alkyl, (C₂-C₆)-alkenyl, (C₂-C₆)-alkynyl, fluorine, chlorine, bromine, iodine, (C₁-C₃)-haloalkyl, (C₃-C₆)-cycloalkyl, heteroaryl, carboxyl and ester.

3. Method according to claim 2, **characterised in that** the further substituent is selected from: methyl, ethyl, fluoro, chloro, bromo, iodo, carboxyl groups and two or more thereof.

4. Method according to one of claims 1 to 3, **characterised in that** R¹ and R² are independently selected from the group consisting of methyl, ethyl, *n-*propyl, isopropyl, *n*-butyl, isobutyl, *tert*-butyl, *n*-pentyl, isopentyl, *tert*-pentyl.

5. Method according to claim 4, **characterised in that** R¹ stands for methyl or ethyl and R² stands for ethyl or isobutyl, preferably R¹ stands for methyl and R² stands for isobutyl or R¹ and R² stand for ethyl.

6. Method according to claim 5, **characterised in that** methyl isobutyl ketone or 3-pentanone, preferably methyl isobutyl ketone, is used as the ketone of formula (I).

7. Method according to one or more of claims 1-6, **characterised in that** the concentration of the mineral acid amounts from approximately 10 to approximately 98%, preferably from approximately 20 to approximately 50% or from approximately 95 to approximately 97%.

8. Method according to one or more of claims 1-7, **characterised in that** the ratio of amount of the organic solvent of formula (I) to the aqueous solution of the mineral acid amounts to approximately 1:100 to approximately 100:1, preferably approximately 1:25 to approximately 25:1.

9. Method according to one or more of claims 1-8, **characterised in that** sulphuric acid or phosphoric acid, preferably sulphuric acid, is used as a mineral acid.

10. Method according to one or more of claims 1-9, **characterised in that** the aryldiazonium salt is decomposed at a temperature of approximately 70 to approximately 120°C, preferably from approximately 80 to approximately 110°C.

## Revendications

1. Procédé pour la préparation de phénols, dans lequel un sel d'aryldiazonium, qui est préparé par diazotation d'une amine primaire correspondante, est décomposé par chauffage dans un mélange contenant de l'eau chaude, un acide minéral et un solvant organique, **caractérisé en ce que** le solvant organique contient une cétone de formule (I) R¹C(O)R² 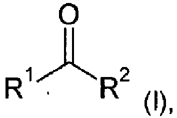 dans laquelle R¹ et R² représentent, indépendamment l'un de l'autre, (C₁-C₅)-alkyle et R¹ et R² présentent, ensemble, au moins quatre atomes de carbone, l'amine primaire aromatique étant l'aniline ou une aniline substituée, qui contient au moins un autre substituant, qui est choisi parmi: alkyle, alcényle, alcynyle, halogène, halogénoalkyle, cycloalkyle, hétéroaryle, carboxyle, cyano, alcoxyle et ester, et le mélange contenant de l'eau chaude, un acide minéral et un solvant organique ne contenant pratiquement pas de sels de cuivre.

2. Procédé selon la revendication 1, **caractérisé en ce que** l'aniline contient au moins un autre substituant, qui est choisi parmi: (C₁-C₆)-alkyle, (C₂-C₆)-alcényle, (C₂-C₆)-alcynyle, fluor, chlore, brome, iode, (C₁-C₃)-halogénoalkyle, (C₃-C₆)-cycloalkyle, hétéroaryle, carboxyle et ester.

3. Procédé selon la revendication 2, **caractérisé en ce que** l'autre substituant est choisi parmi : les groupes méthyle, éthyle, fluor, chlore, brome, iode, carboxyle ou deux ou plus de ceux-ci.

4. Procédé selon l'une quelconque des revendications 1 à 3, **caractérisé en ce que** R¹ et R² sont choisis, indépendamment l'un de l'autre, dans le groupe constitué par méthyle, éthyle, n-propyle, isopropyle, n-butyle, isobutyle, tert-butyle, n-pentyle, isopentyle, tert-pentyle.

5. Procédé selon la revendication 4, **caractérisé en ce que** R¹ représente méthyle ou éthyle et R² représente éthyle ou isobutyle, de préférence R¹ représente méthyle et R² représente isobutyle ou R¹ et R² représentent éthyle.

6. Procédé selon la revendication 5, **caractérisé en ce qu'**on utilise de la méthylisobutylcétone ou du 3-pentanone, de préférence de la méthylisobutylcétone, comme cétone de formule (I).

7. Procédé selon l'une ou plusieurs des revendications 1-6, **caractérisé en ce que** la concentration de l'acide minéral est d'environ 10 à environ 98%, de préférence d'environ 20 à environ 50% ou d'environ 95 à environ 97%.

8. Procédé selon l'une ou plusieurs des revendications 1-7, **caractérisé en ce que** le rapport des quantités de solvant organique de formule (I) et de la solution aqueuse de l'acide minéral est d'environ 1:100 à environ 100:1, de préférence d'environ 1:25 à environ 25:1.

9. Procédé selon l'une ou plusieurs des revendications 1-8, **caractérisé en ce qu'**on utilise de l'acide sulfurique ou de l'acide phosphorique, de préférence de l'acide sulfurique, comme acide minéral.

10. Procédé selon l'une ou plusieurs des revendications 1-9, **caractérisé en ce que** le sel d'aryldiazonium est décomposé à une température d'environ 70 à environ 120°C, de préférence d'environ 80 à environ 110°C.
